# EUROPÄISCHE PATENTSCHRIFT

(11) **EP 3 593 767 B1**
(45) Veröffentlichungstag und Bekanntmachung des Hinweises auf die Patenterteilung: **31.08.2022**
(21) Anmeldenummer: 19185106.2
(22) Anmeldetag: 09.07.2019
(51) Int. Cl.: A61F 5/01, A61F 5/058, A61F 5/37, B33Y 80/00, A61F 5/055

(54) **ORTHESE MIT ZUMINDEST EINEM SCHALENARTIGEN STÜTZTEIL**
ORTHESIS WITH AT LEAST ONE SHELL-SHAPED SUPPORT PART
ORTHÈSE DOTÉE D'AU MOINS UNE PARTIE DE SUPPORT EN FORME DE COQUILLE

(30) Priorität: 12.07.2018 DE 102018116872
(43) Veröffentlichungstag der Anmeldung: 15.01.2020
(73) Patentinhaber: Efinger Orthopädietechnik GmbH, 97074 Würzburg (DE); Sternkopf, Sönke, 97249 Eisingen (DE)
(72) Erfinder: STERNKOPF, Sönke, 97249 Eisingen (DE)
(74) Vertreter: advotec.

(56) Entgegenhaltungen:
- EP-A1- 2 319 967
- WO-A1-2015/006766
- WO-A1-2017/042550
- CN-A- 106 214 308
- DE-A1-102016 207 651
- DE-U1-202013 101 385
- US-A- 3 374 785
- US-B1- 7 018 351

## Beschreibung

Die Erfindung betrifft eine Orthese mit zumindest einem schalenartigen Stützteil nach dem Oberbegriff des Anspruchs 1. Insbesondere betrifft die Erfindung eine Kopforthese zur Verhinderung einer lagebedingten Plagiozephalie bei Säuglingen.

Gattungsgemäße Orthesen haben in der Medizin, insbesondere in der Orthopädie, eine weite Verbreitung gefunden und dienen der äußeren Abstützung des knöchernen Stützapparats bei verschiedensten Krankheitsbildern. Kernelement dieser Orthese ist dabei zumindest ein schalenartiges Stützteil, das am entsprechenden Körperteil abstützend angelegt und dort lösbar fixiert werden kann. Durch die harte Schale des Stützteils kann das Körperteil von außen her entlastet und entsprechend geeignet therapiert werden.

Um einen entsprechend geeigneten Tragekomfort für den Benutzer der Orthese zu ermöglichen, wird auf der zum Körperteil weisenden Innenseite des Stützteils der gattungsgemäßen Orthese zumindest bereichsweise ein Bezug, beispielsweise ein geeignetes Stoffgewebe, befestigt.

Durch den Bezug kann Körperfeuchtigkeit aufgenommen und die Haut auch bei längerem Tragen der Orthese zumindest geringfügig belüftet werden.

Insbesondere, jedoch keineswegs ausschließlich, finden gattungsgemäße Orthesen zur therapeutischen Behandlung von Plagiozephalien oder brachyzephalen Schädelverformungen an Hinterköpfen von Säuglingen Verwendung. Diese speziellen Orthesen sind in der Art von Kopforthesen gestaltet und dienen der äußeren Abstützung zumindest eines Teilbereichs der Schädelknochen. Bei diesem Krankheitsbild können asymmetrische, nicht gleichmäßig abgerundete gewölbte Kopfverformungen bei Säuglingen durch Lageanomalien im Mutterleib oder auch bei der Geburt verursacht worden sein. Diese Verformungen können nach der Geburt bei ständiger Rückenlagerung weiter verstärkt werden. Auch ist es denkbar, dass Kopfverformungen durch übermäßige Rückenlagerung der Säuglinge nach der Geburt verursacht werden, wenn der Kopf des Säuglings in seinen ersten Lebensmonaten bevorzugt auf der abgeflachten Hinterkopfseite liegt.

Plagiozephale einseitige Schädelabflachungen können zu einseitigen Vorzugshaltungen der Köpfe der Säuglinge zu einer Seite hin führen. Durch die einseitige Vorzugshaltung der Köpfchen der Säuglinge entwickelt sich folgerichtig eine asymmetrische Ganzkörperhaltung der Säuglinge mit nur einseitigen Gleichgewichtsreaktionen. Es kommt dadurch verursacht zu einer funktionellen Dysbalance zwischen der rechten und linken Körperhälfte.

Zur Behandlung dieser plagiozephalen einseitigen Schädelabflachungen sind aus der Medizin seit langem Kopforthesen bekannt, die in einer Helmtherapie Verwendung finden. Die bekannten Kopforthesen wurden bisher in aufwendiger Weise nach einem Gipsmodell an die Säuglingsköpfe angepasst. Diese Herstellung der Kopforthesen auf einem Gipsmodell des Säuglingskopfs war sehr kostenaufwendig und wurde deshalb vielfach erst nach dem sechsten Lebensmonat angewendet. Auch auf die eigentlich notwendige regelmäßige Nachanpassung des am Gipsmodel hergestellten Helms wurde aufgrund des großen Bearbeitungsaufwands vielfach verzichtet. In der Weiterentwicklung dieser bekannten Kopforthesen ist es deshalb bekannt, die Kopforthesen anhand eines Datenmodells des Körperteils, nämlich anhand eines gescannten Datenmodells des Säuglingskopfs, mittels eines generativen Herstellungsverfahrens in additiver Fertigung herzustellen. Diese additive Fertigung erfolgt dabei direkt auf der Basis eines rechnerinternen Datenmodels, das beispielsweise durch sensorisches Laserscannen der Kontur des Säuglingsschädels ermittelt wurde. Das Datenmodel wird dann an eine geeignete Fertigungsmaschine, beispielsweise einen 3D-Drucker, übermittelt und das schalenartige Stützteil aus einem formlosen (Flüssigkeiten, Gele, Pasten, Pulver o.ä.) oder formneutralen (bandförmigen, drahtförmigen oder blattförmigen) Material mittels chemischer und/oder physikalischer Prozesse additiv hergestellt. Auf die Herstellung eines Modells oder einer Form zur Herstellung des Stützteils kann bei diesen generativen Herstellungsverfahren zur additiven Fertigung aus einem formlosen bzw. formneutralen Material verzichtet werden, was die Fertigungskosten signifikant verringert.

Durch die Verwendung generativer Fertigungsverfahren zur additiven Fertigung des schalenartigen Stützteils werden die Fertigungskosten zur Fertigung des Stützteils massiv gesenkt. Andererseits hat die generative Fertigung des Stützteils aber den Nachteil, dass bei der Fertigung der Kopforthese kein Modellkopf mehr zur Verfügung steht. Dieses Fehlen eines Modells zur Herstellung der Orthese ist insbesondere von Nachteil, wenn auf der Innenseite des Stützteils ein Bezug vorgesehen werden soll. Denn bei der traditionellen Fertigung von Orthesen unter Verwendung eines Modells wurde die Orthese schichtweise auf dem Modell aufgebaut, wobei in der ersten Schicht der Bezug auf das Modell aufgelegt und zugeschnitten wurde. Angesichts des Fehlens eines Modells des Körperteils bei Fertigung der gattungsgemäßen Orthesen kann diese Art der Fertigung des Bezugs nicht mehr eingesetzt werden. Vielmehr muss bei additiver Herstellung des schalenartigen Stützteils der Bezug nach Fertigstellung des Stützteils auf der Innenseite des Stützteils angepasst, zugeschnitten und befestigt werden. Diese Art der Herstellung und Befestigung des Bezugs an dem schalenartigen Stützteil ist außerordentlich aufwendig und wenig exakt.

Aus der US 7,018,351 B1 ist eine Unterschenkelorthese mit einem mehrschichtigen Aufbau bekannt. Auf der Innenseite einer harten Schale wird eine weichere Innenschicht angeordnet, deren Oberfläche wiederum mit einem Bezug versehen sein kann. Zur Befestigung der Innenschicht und des daran befestigten Bezugs ist ein Befestigungsprofil am Rand der Schale angebracht.

Die WO2017/042550 A1 beschreibt eine Kopforthese mit mehrschichtigen Aufbau. In einer aus Kunststoff hergestellten Stützschale ist ein 3D-Maschengewebe angeordnet, das mit einem additiven Herstellungsverfahren, nämlich im 3D-Druck aus Kunststoff hergestellten ist. Das ein 3D-Maschengewebe wird dabei individuell auf die Kopfform des Patienten angepasst und entsprechend patientenbezogen gefertigt. Auf der Innenseite des ein 3D-Maschengewebe ist wiederum ein Bezug angeordnet, der mit Klettverschlusselementen fixiert wird.

Nachteilig an der Befestigung des Bezuges mit den Klettverschlusselementen ist es, dass die Schnittkanten am Rand des Bezuges offen liegen und nicht überdeckt werden. Das Bezugsgewebe kann dadurch entlang der Schnittkante leicht ausfasern, wodurch der Tragekomfort und die Hygiene der Orthese beeinträchtigt wird.

Aufgabe der vorliegenden Erfindung ist es deshalb, eine Orthese, insbesondere eine Kopforthese, vorzuschlagen, deren schalenartiges Stützteil auf der Innenseite mit einem Bezug ausgestattet ist, der einfach und kostengünstig an dem Stützteil befestigt werden kann und dabei die Nachteile des vorbekannten Stand der Technik vermeidet.

Diese Aufgabe wird durch eine Orthese nach der Lehre des Anspruchs 1 gelöst.

Vorteilhafte Ausführungsformen der Erfindung sind Gegenstand der Unteransprüche.

Die erfindungsgemäße Orthese beruht auf dem Grundgedanken, dass am Rand des Stützteils und/oder auf der Außenseite des Stützteils zumindest ein Befestigungselement additiv angeformt ist, an dem der Rand des Bezugs befestigt werden kann. Denn die zusätzliche Anformung des erfindungsgemäßen Befestigungselements am Rand bzw. an der Außenseite des Stützteils ist bei Herstellung des Stützteils unter Verwendung eines generativen Herstellungsverfahrens in additiver Fertigung problemlos möglich. Dazu muss lediglich die Modellierung des additiv anzuformenden Befestigungselements zusätzlich in das Datenmodell aufgenommen und entsprechend ergänzt werden. Beim Herstellen des Stützteils wird dann das Befestigungselement ohne signifikanten Mehraufwand in bekannter Weise an das schalenartige Stützteil mit angeformt. Durch die erfindungsgemäße Ergänzung der Orthese mit dem Befestigungselement am Rand oder an der Außenseite des Stützteils kann die nachträgliche Befestigung des Bezugs auf der Innenseite des Stützteils massiv vereinfacht werden. Denn der Bezug kann durch das zusätzliche Befestigungselement problemlos am Rand des Stützteils oder an der Außenseite des Stützteils befestigt werden. Auf diese Weise werden die Herstellungskosten zur Herstellung entsprechender Orthesen mit innenseitigem Bezug signifikant gesenkt.

Eine besonders einfache und effektive Befestigung des Bezugs am Stützteil wird ermöglicht, wenn das Befestigungselement in der Art eines Befestigungsprofils bzw. in der Art einer Befestigungsnut ausgebildet ist. Das Befestigungsprofil bzw. die Befestigungsnut kann sich dabei entweder entlang der gesamten Kontur des Bezugsrandes oder auch nur abschnittsweise entlang des Bezugsrandes erstrecken. Durch das Befestigungsprofil bzw. die Befestigungsnut werden die zur Verfügung stehenden Befestigungsmittel zur Befestigung des Bezugsrandes am Stützteil stark vereinfacht. Außerdem kann der vielfach aufgrund des Zuschnitts optisch nicht optimale Bezugsrand im Befestigungsprofil bzw. in der Befestigungsnut nach außen hin optisch überdeckt werden, um so eine optimale Optik der Orthese zu erreichen. Erfindungsgemäß ist am Stützteil eine Klebstoffnut vorgesehen, in der Klebstoff aufgenommen werden kann. Zur Herstellung des Bezugs wird der Rand des Bezugs in diese Klebstoffnut eingeführt und anschließend durch Zuführung von Klebstoff in der Klebstoffnut fixiert. Das Einkleben des Bezugsrandes in die Klebstoffnut führt zu einer hochfesten Verbindung zwischen Bezug und Stützteil bei optimaler Optik. Um eine Nachbearbeitung des Randes des Stützteils zur Anpassung an das Körperteil zu ermöglichen, ist es besonders vorteilhaft, wenn das auf der Außenseite des Stützteils angeordnete Befestigungselement einen Abstand zum Rand des Stützteils aufweist. Dieser Abstand kann dann während der Nachbearbeitung der Kontur des Stützteils teilweise oder ganz entfernt werden, ohne dass die Befestigungsmöglichkeit zur Befestigung des Bezugsrandes beeinträchtigt wird.

Wie bereits erwähnt, ist für den Tragekomfort eine ausreichende Belüftung der Haut auf der Innenseite des Stützteils von großer Bedeutung. Dies ist insbesondere dann zu bevorzugen, wenn die Orthese in der Art einer Kopforthese für einen Säugling ausgebildet ist, da die Säuglingshaut sehr empfindlich ist und die Kopforthesen für viele Stunden am Tag getragen werden müssen. Um einen hohen Belüftungsfaktor zu realisieren, ist es deshalb von großem Vorteil, wenn das Stützteil eine Vielzahl von Belüftungslöchern aufweist. Durch die additive Fertigung des Stützteils können diese Belüftungslöcher ohne maßgeblichen Mehraufwand problemlos realisiert werden.

Im Hinblick auf den wünschenswerten Belüftungskomfort ist es weiterhin besonders vorteilhaft, wenn der Bezug aus einem luftdurchlässigen Bezugsstoff hergestellt ist.

Im Hinblick auf die formgerechte Fixierung des Bezugs auf der Innenseite des Stützteils ist es vorteilhaft, wenn der Bezug der erfindungsgemäßen Orthese aus einem in der Bezugsebene zweiachsig verformbaren Bezugsstoff hergestellt ist. Durch diese zweiachsige Verformbarkeit des Bezugsstoffs kann der zunächst unverformte Bezugsstoff problemlos an die Konturen auf der Innenseite des Stützteils angepasst und dort befestigt, beispielsweise angeklebt, werden.

Aus welchem Material die Orthese hergestellt ist, ist grundsätzlich beliebig. Besonders einfach und kostengünstig kann das generative Herstellungsverfahren zur additiven Fertigung der erfindungsgemäßen Orthese bei Verwendung von Kunststoff eingesetzt werden. Zur Herstellung von erfindungsgemäßen Orthesen ist es vorgesehen, ein Stützteil anhand einen Datenmodells des Körperteils mit einem generativen Herstellungsverfahren in additiver Fertigung herzustellen, wobei am Rand des Stützteils und/oder auf der Außenseite des Stützteils zumindest ein Befestigungselement additiv angeformt ist, um daran den Rand des Bezugs der Orthese befestigen zu können.

Verschiedene Ausführungsformen der Erfindung sind in den Zeichnungen schematisiert dargestellt und werden nachfolgend beispielhaft erläutert. Es zeigen:
- **Fig. 1**: eine erfindungsgemäße Orthese, nämlich eine Kopforthese zur Verhinderung einer lagebedingten Plagiozephalie, in perspektivischer Ansicht von oben;
- **Fig. 2**: die Kopforthese gemäß Fig. 1 in Ansicht von hinten;
- **Fig. 3**: die Kopforthese gemäß Fig. 1 in seitlicher Ansicht;
- **Fig. 4**: die Kopforthese gemäß Fig. 3 im Teilquerschnitt entlang der Schnittlinie I-I;
- **Fig. 5**: das in Fig. 4 im Querschnitt dargestellte Befestigungselement des Stützteils nach Befestigung des Bezugs durch Einbringen eines Klebstoffs;

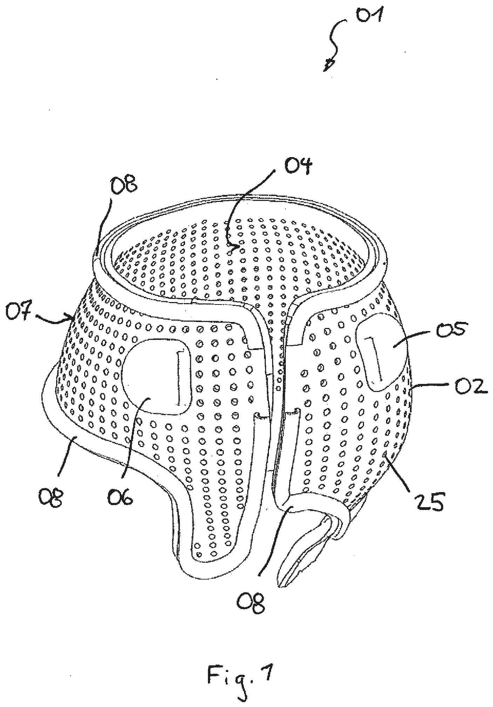

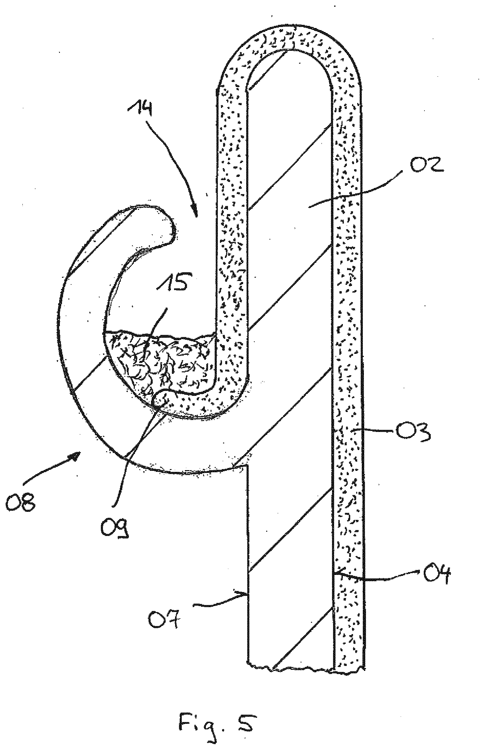

**Fig. 1** zeigt eine erfindungsgemäße Orthese, nämlich eine Kopforthese 01 zur Verhinderung einer lagebedingten Plagiozephalie, in perspektivischer Ansicht. Fig. 1 zeigt dabei lediglich das Stützteil 02 der Kopforthese 01 ohne Anbauteile. Zur Vervollständigung der Kopforthese 01 ist an dem Stützteil 02 insbesondere noch ein Bezug 03 (siehe Fig. 5) auf der Innenseite 04 des Stützteils 03 zu befestigen. Außerdem werden an den beiden Sockelelementen 05 und 06, die in das Stützteil 02 additiv angeformt sind, noch die Enden eines geeigneten Verschlusses befestigt, um die Schlitzung des Stützteils 02 nach dem Anlegen auf dem Säuglingskopf zu verschließen und damit die Kopforthese lagerichtig zu fixieren.

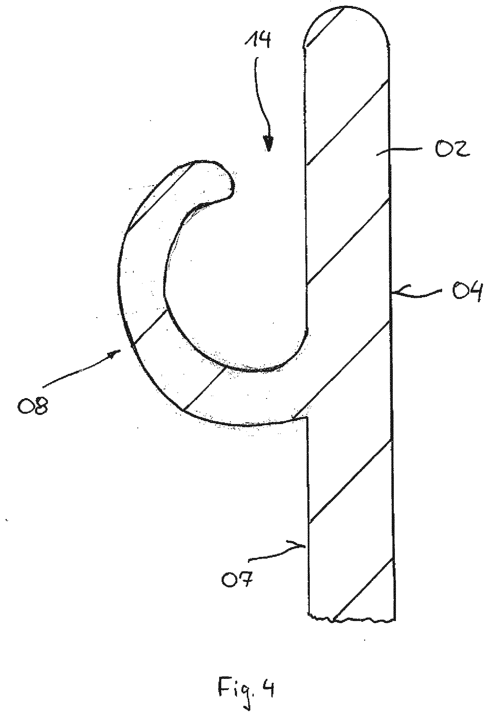

Parallel zur Kontur des Randes des Stützteils 02 ist auf der Außenseite 07 des Stützteils 02 abschnittsweise ein Befestigungselement 08 zur Befestigung des Randes 09 (siehe Fig. 5) des Bezugs 03 vorgesehen. Die Funktion des Befestigungselements 08 wird nachfolgend noch anhand der Darstellungen in Fig. 4 und Fig. 5 näher erläutert.

Die Kopforthese 01 weist eine Vielzahl von Belüftungslöchern 25 in dem Stützteil 02 auf, um den Belüftungskomfort bei der Kopforthese 01 zu verbessern.

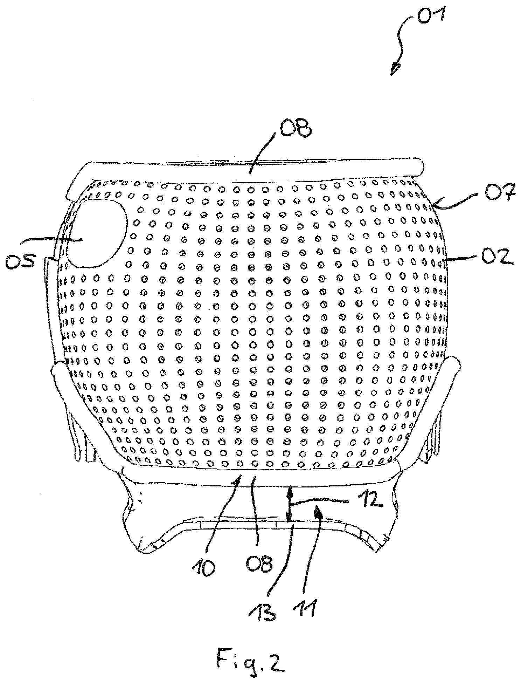

**Fig. 2** zeigt die Kopforthese 01 in Ansicht von hinten. Man erkennt, dass der Abschnitt 10 des Befestigungselements 08 im Bereich des Nackenabschnitts 11 des Stützteils 02 einen in der Länge variierenden Abstand 12 zum Rand 13 des Stützteils 02 aufweist. Durch diesen Abstand 12 wird es ermöglicht, dass bei der Anpassung der Kopforthese 01 an den Säuglingskopf eine Nachbearbeitung des Randes 13 im Nackenabschnitt 12 ermöglicht wird, ohne dass dabei der Abschnitt 12 des Befestigungselements 08 beeinträchtigt wird.

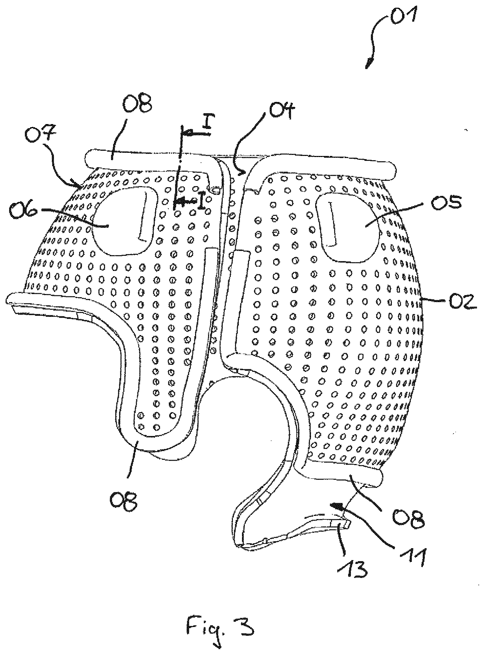

**Fig. 3** zeigt die Kopforthese 01 in seitlicher Ansicht. Die Funktionalität des Befestigungselements 08 soll nachfolgend anhand eines Teilquerschnitts entlang der Schnittlinie I-I näher erläutert werden.

**Fig. 4** zeigt das Stützteil 02 mit dem Befestigungselement 08 im Teilquerschnitt entlang der Schnittlinie I-I. Das Befestigungselement 08 ist in der Art einer Klebstoffnut 14 ausgebildet, in der der Rand 09 eines Bezugs 03 befestigt werden kann.

**Fig. 5** zeigt das Stützteil 02 nach Anbringung des Bezugs 03 und nach Befestigung des Randes 09 in der Klebstoffnut 14. Ausgehend von der Innenseite 04 wird der Bezug 03 über den Rand 13 des Stützteils 02 gezogen und verläuft auf der Außenseite 07 bis an den Grund der Klebstoffnut 14. Bei der Montage der Kopforthese 01 wird der Rand 09 dann durch Einbringung eines Klebstoffs 15 in die Klebstoffnut 14 mit dem Stützteil 02 adhäsiv verbunden und fixiert.

## Patentansprüche

1. Orthese (01) mit zumindest einem schalenartigen Stützteil (02), das an einem Körperteil abstützend angelegt und lösbar fixiert werden kann, wobei das Stützteil (02) anhand eines Datenmodels des Körperteils mit einem generativen Herstellungsverfahren in additiver Fertigung hergestellt ist, und wobei auf der zum Körperteil weisenden Innenseite (04) des Stützteils (02) zumindest bereichsweise ein Bezug (03) angeordnet ist,
**dadurch gekennzeichnet,**
**dass** am Rand des Stützteils und/oder auf der Außenseite (07) des Stützteils (02) zumindest ein Befestigungselement (08), an dem der Rand (09) des Bezugs (03) befestigt werden kann, additiv angeformt ist, wobei das Befestigungselement (08) in der Art einer Klebstoffnut (14), in der ein Klebstoff (15) aufgenommen werden kann, ausgebildet ist, wobei der Rand (09) des Bezugs (03) mit dem Klebstoff (15) in der Klebstoffnut (14) fixiert wird.

2. Orthese nach Anspruch 1,
**dadurch gekennzeichnet,**
**dass** zwischen dem Rand (13) des Stützteils (02) und dem auf der Außenseite (07) des Stützteils (02) angeordneten Befestigungselement (08) zumindest abschnittsweise ein Abstand (12) vorhanden ist.

3. Orthese nach Anspruch 1 oder 2,
**dadurch gekennzeichnet,**
**dass** das Stützteil (02) eine Vielzahl von Belüftungslöchern (25) aufweist.

4. Orthese nach einem der Ansprüche 1 bis 3,
**dadurch gekennzeichnet,**
**dass** der Bezug (03) aus einem luftdurchlässigen Bezugsstoff hergestellt ist.

5. Orthese nach einem der Ansprüche 1 bis 4,
**dadurch gekennzeichnet,**
**dass** der Bezug (03) aus einem in der Bezugsebene zweiachsig verformbaren Bezugsstoff hergestellt ist.

6. Orthese nach einem der Ansprüche 1 bis 5,
**dadurch gekennzeichnet,**
**dass** das Stützteil (02) mit dem generativen Herstellungsverfahren in additiver Fertigung aus Kunststoff hergestellt ist.

7. Orthese nach einem der Ansprüche 1 bis 6,
**dadurch gekennzeichnet,**
**dass** die Orthese in der Art einer Kopforthese (01) zur Verhinderung einer lagebedingten Plagiozephalie ausgebildet ist.

## Claims

1. An orthosis (01) having at least one shell-like support part (02) which is capable of being put on a body part in a supporting manner and of being detachably fixed on said body part, the support part (02) being manufactured on the basis of a data model of the body part using a generative manufacturing process in additive manufacturing, and a cover (03) being arranged at least in some areas on the inside (04) of the support part (02) oriented towards the body part,
**characterized in that**
at least one attachment element (08) to which the edge (09) of the cover (03) is fixable is formed additively on the edge of the support part and/or on the outside (07) of the support part (02), the attachment element (08) being realized in the manner of an adhesive groove (14) in which an adhesive (15) is capable of being accommodated, the edge (09) of the cover (03) being fixed in the adhesive groove (14) with the adhesive (15).

2. The orthosis according to claim 1,
**characterized in that**
a distance (12) is present at least sectionally between the edge (13) of the support part (02) and the attachment element (08) arranged on the outside (07) of the support part (02).

3. The orthosis according to claim 1 or 2,
**characterized in that**
the support part (02) has a plurality of vent holes (25).

4. The orthosis according to any one of claims 1 to 3,
**characterized in that**
the cover (03) is made of a cover fabric permeable to air.

5. The orthosis according to any one of claims 1 to 4,
**characterized in that**
the cover (03) is made of a cover fabric which is biaxially deformable in the cover plane.

6. The orthosis according to any one of claims 1 to 5,
**characterized in that**
the support part (02) is made of plastic by means of a generative manufacturing process in additive manufacturing.

7. The orthosis according to any one of claims 1 to 6,
**characterized in that**
the orthosis is realized in the manner of a cranial orthosis (01) for preventing a positional plagiocephaly.

## Revendications

1. Orthèse (01) ayant au moins une partie de support (02) en forme de coque qui est capable d'être mise sur une partie du corps de manière à supporter ladite partie de corps et d'y être fixée de manière amovible, la partie de support (02) étant fabriquée sur la base d'un modèle de données de la partie du corps en utilisant un procédé de fabrication génératif dans une fabrication additive, et un revêtement (03) étant disposé au moins par endroits sur le côté intérieur (04) de la partie de support (02) qui est orienté vers la partie du corps,
**caractérisée en ce**
**qu'**au moins un élément d'attache (08) sur lequel la bordure (09) du revêtement (03) est fixable est formé additivement sur le bord de la partie de support et/ou sur le côté extérieur (07) de la partie de support (02), l'élément d'attache (08) étant réalisé à la manière d'une rainure d'adhésif (14) dans laquelle de l'adhésif (15) est capable d'être logé, la bordure (09) du revêtement (03) étant fixée dans la rainure d'adhésif (14) avec ledit adhésif (15).

2. Orthèse selon la revendication 1,
**caractérisée en ce**
**qu'**une distance (12) est présente au moins par sections entre le bord (13) de la partie de support (02) et l'élément d'attache (08) disposé sur le côté extérieur (07) de la partie de support (02).

3. Orthèse selon la revendication 1 ou 2,
**caractérisée en ce que**
la partie de support (02) a une pluralité de trous de ventilation (25).

4. Orthèse selon l'une quelconque des revendications 1 à 3,
**caractérisée en ce que**
le revêtement (03) est fait d'un tissu de revêtement perméable à l'air.

5. Orthèse selon l'une quelconque des revendications 1 à 4,
**caractérisée en ce que**
le revêtement (03) est fait d'un tissu de revêtement qui est déformable biaxialement dans le plan du revêtement.

6. Orthèse selon l'une quelconque des revendications 1 à 5,
**caractérisée en ce que**
la partie de support (02) est faite de matière plastique au moyen du procédé de fabrication génératif dans une fabrication additive.

7. Orthèse selon l'une quelconque des revendications 1 à 6,
**caractérisée en ce que**
l'orthèse est réalisée à la manière d'une orthèse crânienne (01) pour empêcher une plagiocéphalie positionnelle.
